# EUROPEAN PATENT SPECIFICATION

(11) **EP 2 103 286 B1**
(45) Date of publication and mention of the grant of the patent: **10.10.2012**
(21) Application number: 09250720.1
(22) Date of filing: 13.03.2009
(51) Int. Cl.: A61F 5/00, A61F 2/04

(54) **Implantabel coil for insertion into a hollow body organ**
Implantierbare Spule zum Einsetzen in ein Hohlkörperorgan
Antenne implantable pour insertion dans un organe corporel creux

(30) Priority: 14.03.2008 US 48400
(43) Date of publication of application: 23.09.2009
(73) Proprietor: Ethicon Endo-Surgery, Inc., Cincinnati, OH 45242 (US)
(72) Inventor: Albrecht, Thomas E., Cincinnati Ohio, 45242 (US); Zeiner, Mark S., Mason Ohio, 45040 (US)
(74) Representative: Tunstall, Christopher Stephen

(56) References cited:
- WO-A2-2008/028108
- US-A1- 2004 220 682

## Description

**Field of the Invention**

The present invention has application in conventional open, laparoscopic and endoscopic surgical instrumentation and methods as well application in robotic-assisted surgery. The present invention has even further relation to devices implanted within the stomach to induce weight loss.

**Background of the Invention**

Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of one hundred billion dollars in the United States alone.

A variety of surgical procedures have been developed to treat obesity. The most common currently performed procedure is Roux-en-Y gastric bypass (RYGB). This procedure is highly complex and is commonly utilized to treat people exhibiting morbid obesity. Other forms of bariatric surgery include Fobi pouch, bilio-pancreatic diversion, and gastroplasty or "stomach stapling". In addition, implantable devices are known which limit the passage of food through the stomach and affect satiety.

In view of the highly invasive nature of many of these procedures, efforts have been made to develop less traumatic and less invasive procedures. Gastric-banding is one of these methods. Gastric-banding is a type of gastric reduction surgery attempting to limit food intake by reducing the size of the stomach. In contrast to RYGB and other stomach reduction procedures, gastric-banding does not require the alteration of the anatomy of the digestive tract in the duodenum or jejunum.

However, gastric bands still require invasive surgical techniques. Recently, many new approaches to the treatment of obesity have been described in the art aiming to reduce invasiveness while maintaining effectiveness. First, restrictive procedures aim to reduce the amount of food a person can eat at a given time. One approach is endoscopic gastric restriction, which aims to create a small restrictive pouch in the proximal stomach by fastening anterior and posterior walls of the stomach together, simulating a vertical gastroplasty. Another approach is to use Restrictive sleeves. These are stent like structures, which are placed in the proximal most portion of the stomach and provide a restrictive outlet, preventing patients from overeating. Yet another approach is to use space occupying devices which maintain a constant volume in the stomach, limiting the amount of food a person can ingest at a given time. In yet another approach, physicians use balloons which expand in the stomach. While easy to install and reversible, these devices have been plagued by migration, leading to obstruction. Because of this, they have to be removed within 6 months.

**Summary of the Invention**

An implant for placement within a hollow body organ including a member having an undeployed shape for delivery to the hollow body and a deployed shape for implantation therein. The member has a plurality of links pivotably connected to each other, and a flexible elongated tether connected to the member such that tensioning the tether places the member in the deployed shape. The member has sufficient rigidity in its deployed shape to exert an outward force against an interior of the hollow body so as to bring together two substantially opposing surfaces of the hollow body. The member has an elastomer link connector configured to pull the links together.

**BRIEF DESCRIPTION OF THE DRAWINGS**

FIG. 1 is an endoscopy overtube placed in the esophagus

FIG. 2 is a close up of the stomach with overtube in place

FIG. 3 is an overall view of the implantable coil device

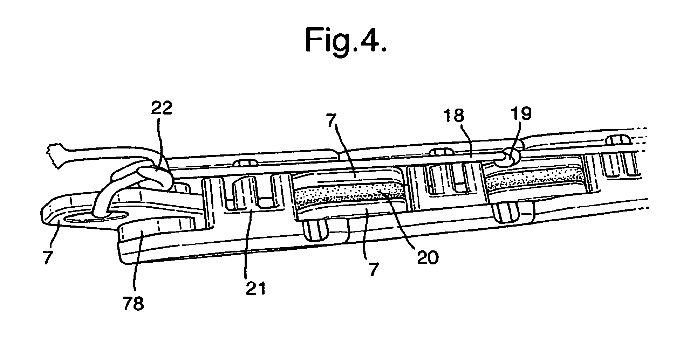

FIG. 4 is a close up of the distal end of the coil

FIG. 5 is a close up of the proximal end of the coil

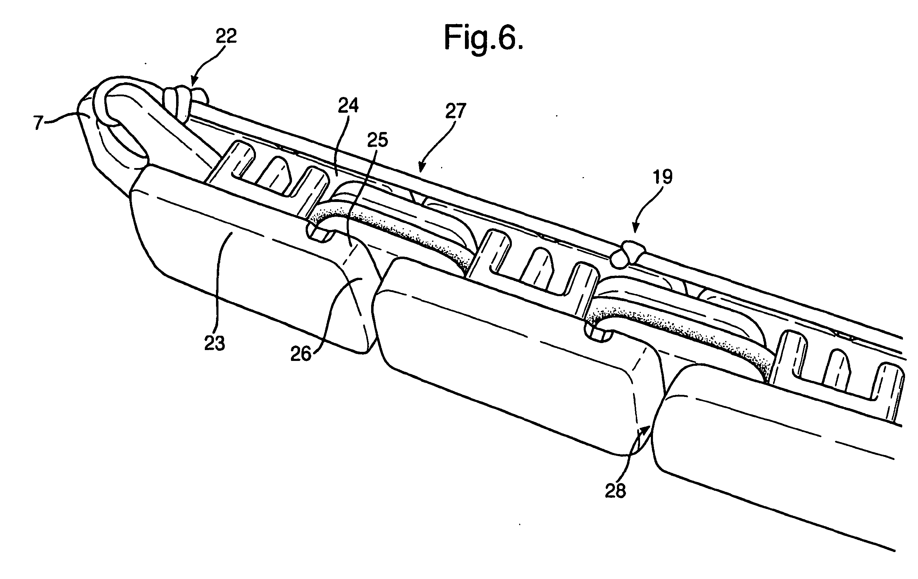

FIG. 6 is an isometric view close up of the distal end of the coil

FIG. 7 is an isometric view of one link with the heat stake assembly

FIG. 8 is an isometric view showing the link assembly in a bent configuration

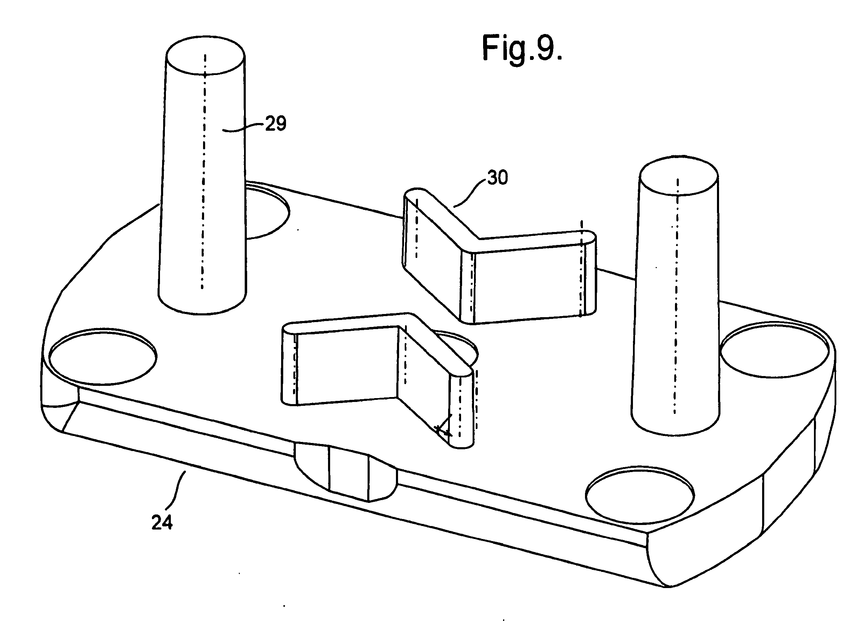

FIG. 9 is an isometric view showing the top link component with the male pins

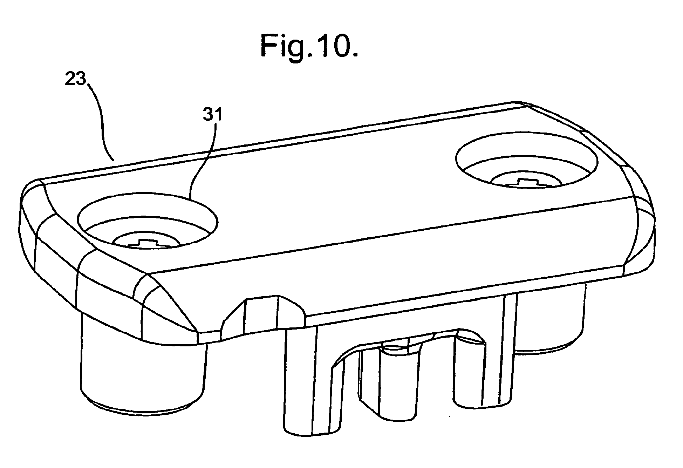

FIG. 10 is an isometric view showing the bottom link component with the counter bore

FIG. 11 is an isometric view showing the bottom link component with pivot boss

FIG. 12 is an cross sectional view showing the pivot pin heat stake assembly

FIG. 13 is various view's showing the individual features on the link assembly

FIG. 14 shows isometric view's of the link connector assembly

FIG. 15 is an exploded view of the proximal tip of the gastric coil

FIG. 16 is a top view of an end of the coil

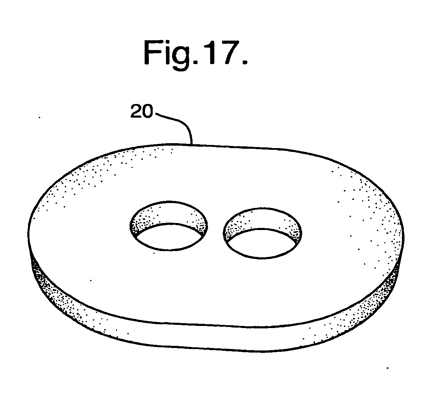

FIG. 17 is a view of the elastomer link

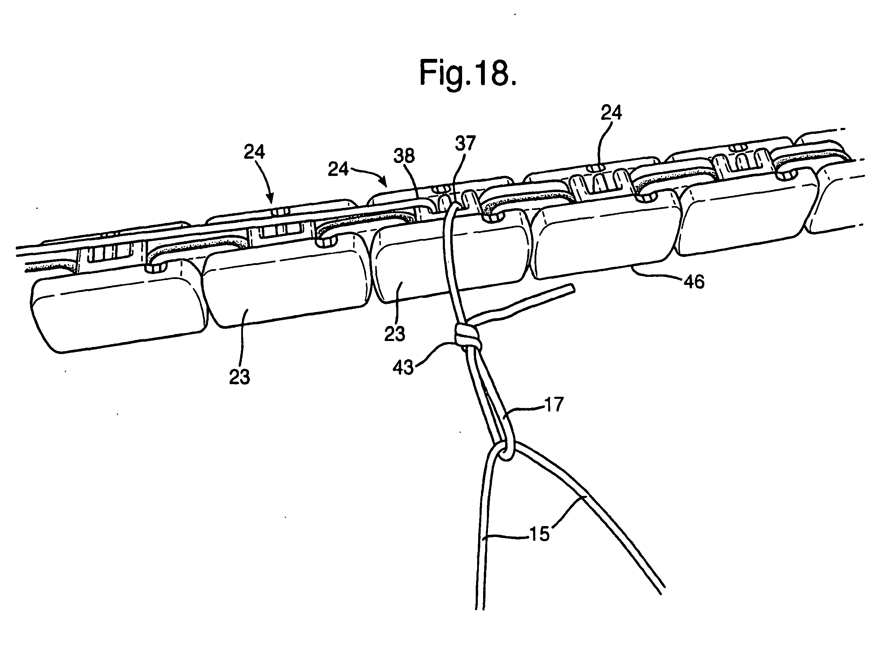

FIG. 18 is a view of the distal string lock with the string configuration shown

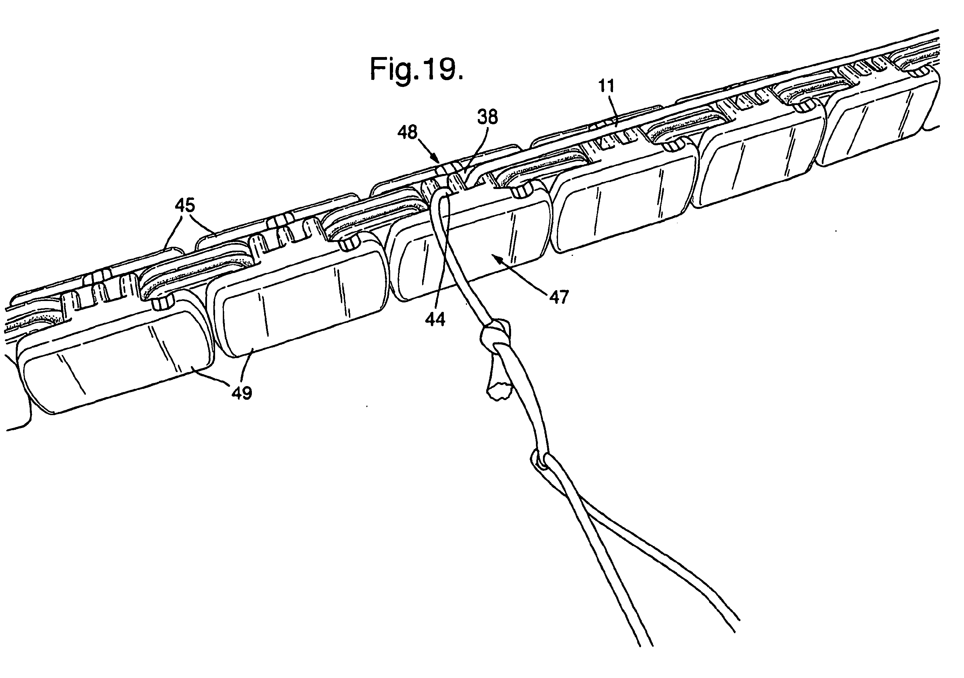

FIG. 19 is a view of the proximal string lock with the string configuration shown

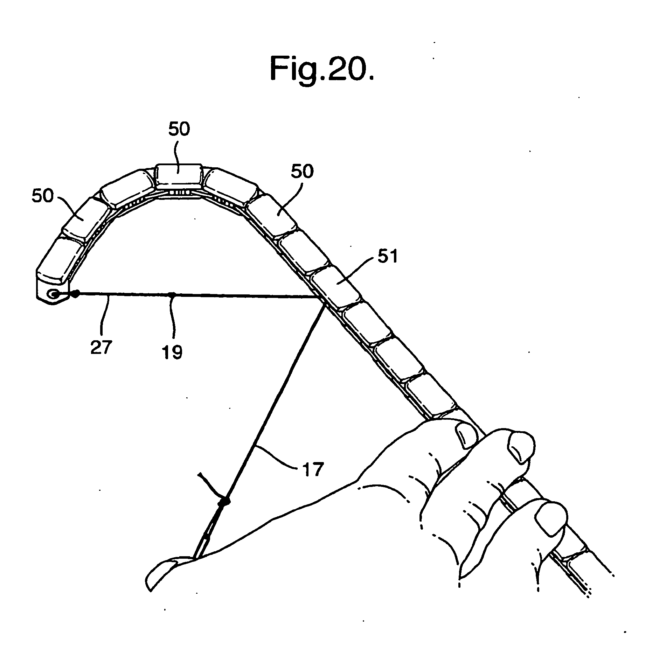

FIG. 20 is a view of the distal string lock with the string configuration pulled into a partially deployment configuration

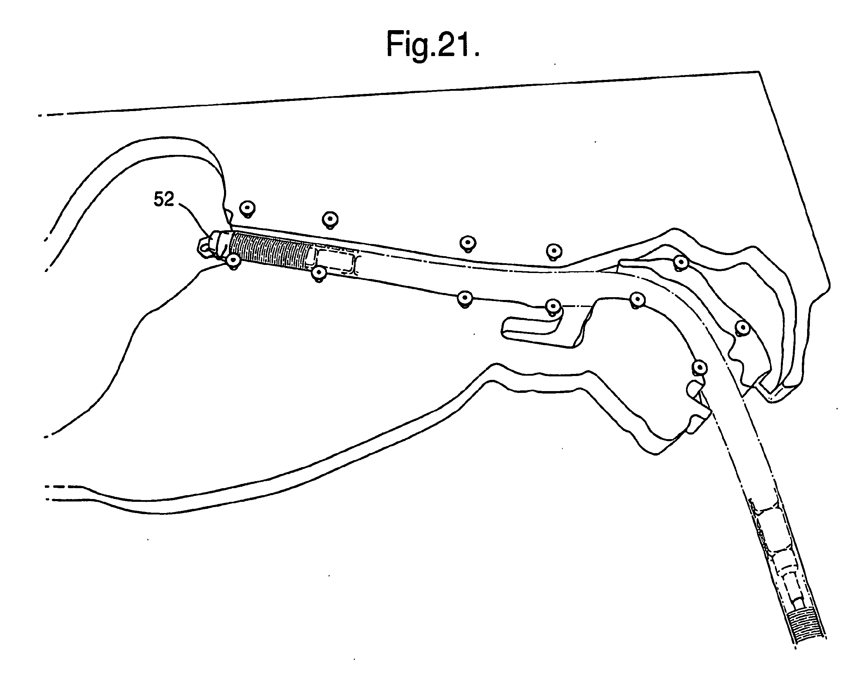

FIG. 21 is a view showing the deployment sequence with the distal tip just entering the stomach

FIG. 22 is a view showing the deployment sequence with the first three links just entering the stomach

FIG. 23 is a view showing the deployment sequence with the first three links just entering the stomach and tension being placed on the pull cable

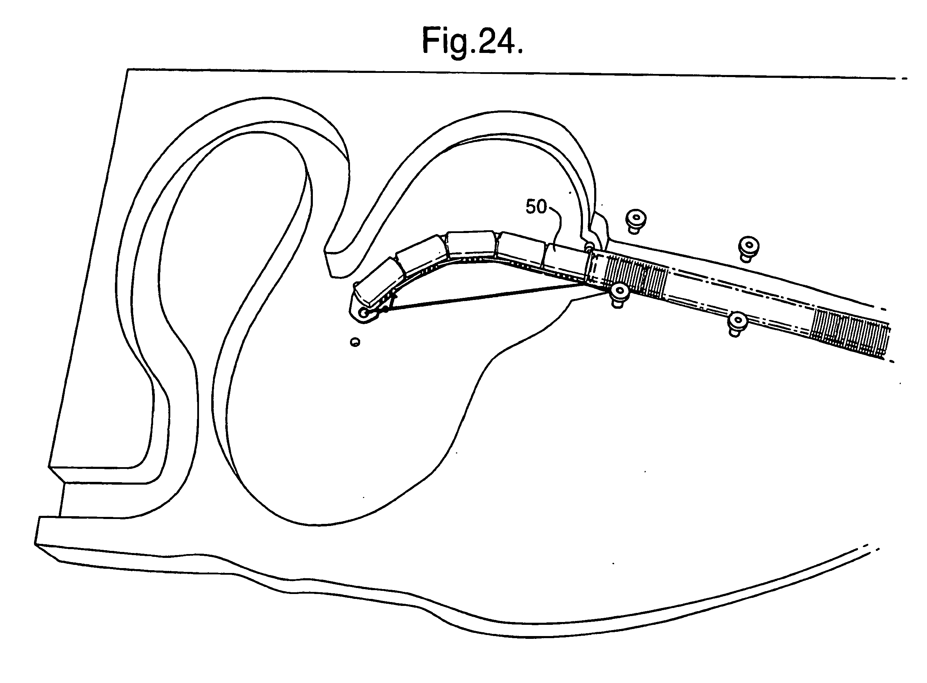

FIG. 24 is a view showing the deployment sequence with the first five links entering the stomach and tension being placed on the pull cable

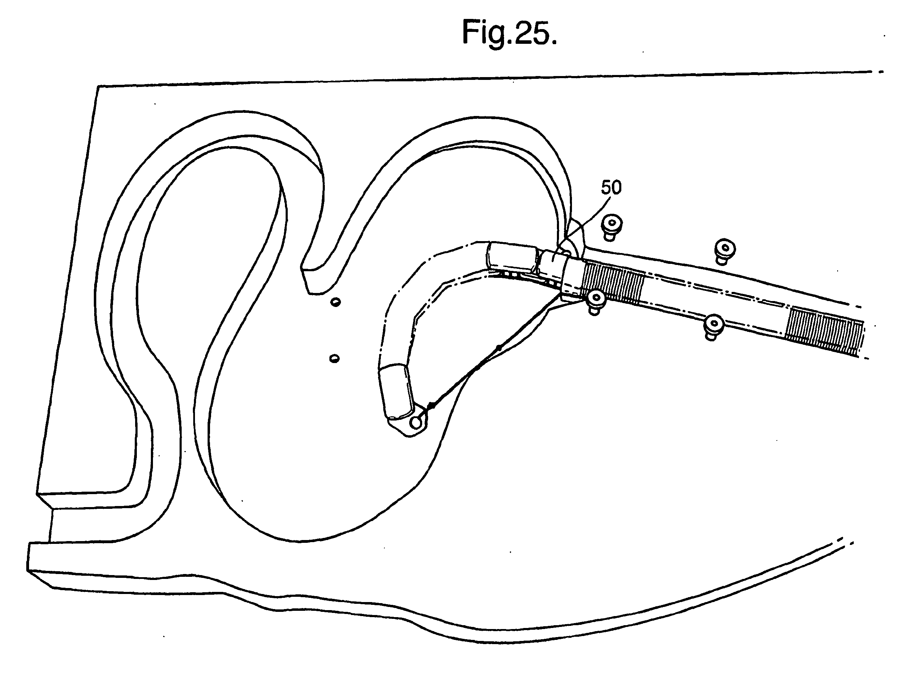

FIG. 25 is a view showing the deployment sequence with the first five links entering the stomach and tension being placed on the pull cable

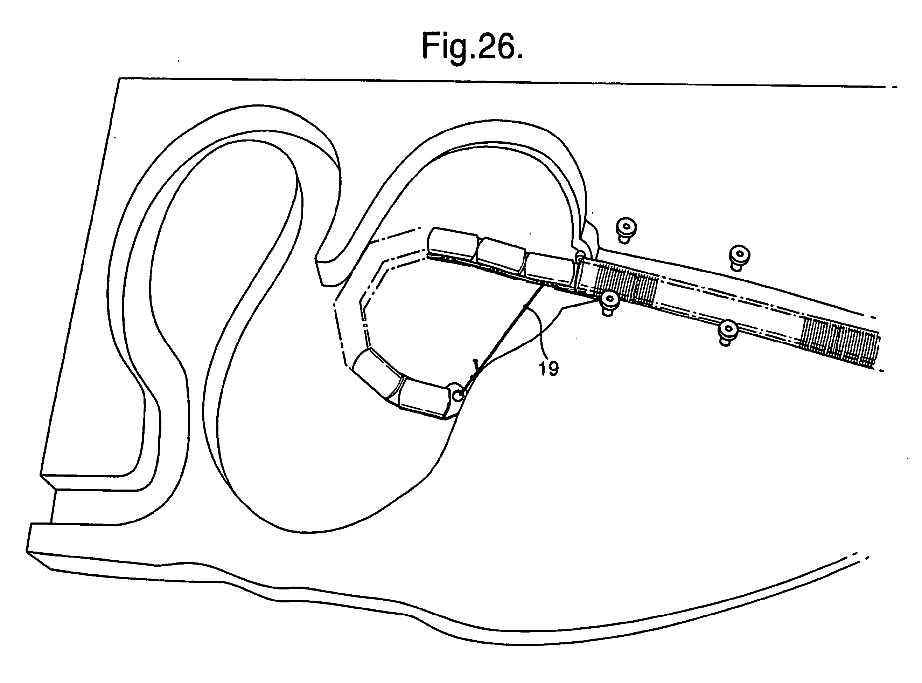

FIG. 26 is a view showing the deployment sequence with the first eight links entering the stomach and tension being placed on the pull cable

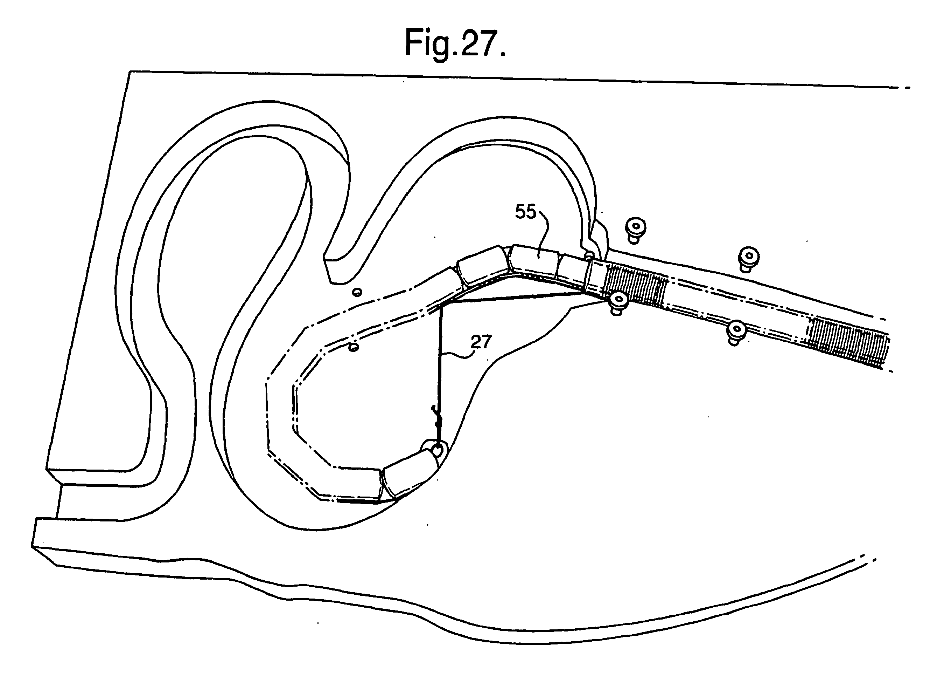

FIG. 27 is a view showing the deployment sequence with the first eleven links entering the stomach and tension being placed on the pull cable

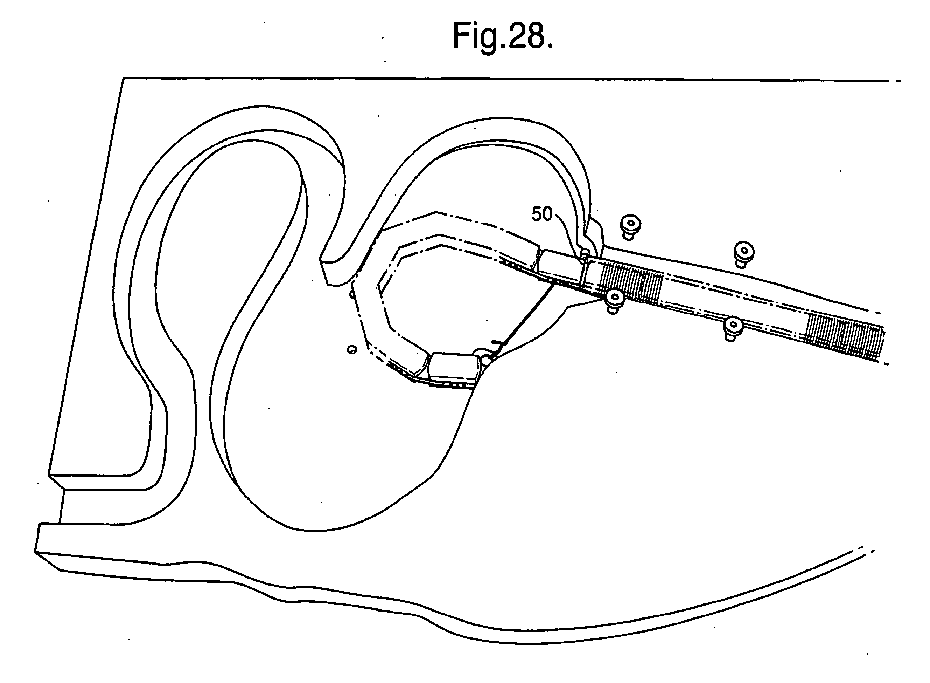

FIG. 28 is a view showing the deployment sequence with the distal end of the coil fully deployed in the stomach with the string lock fully locked into position

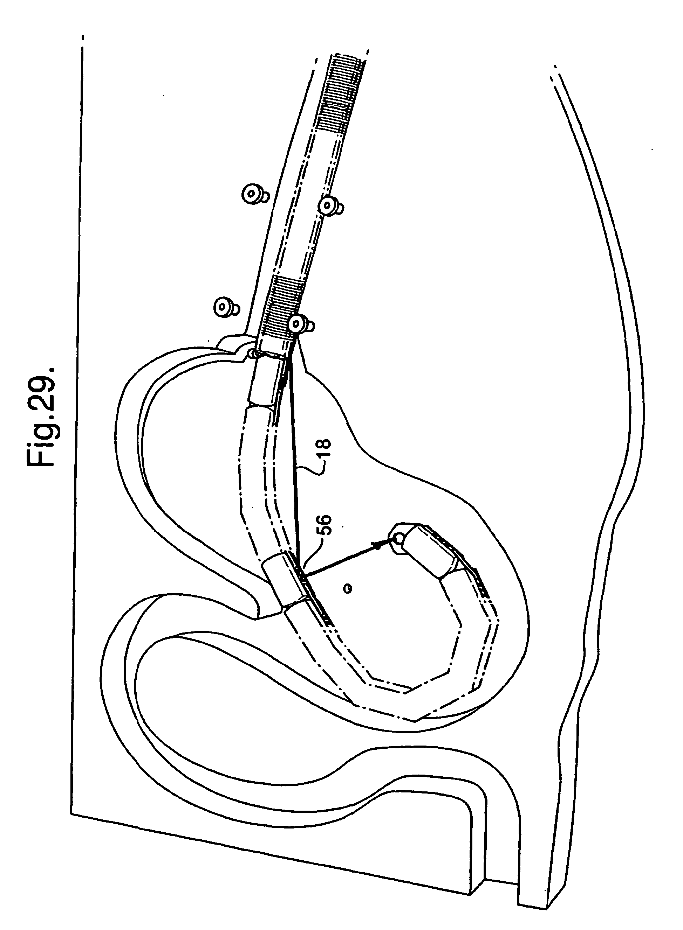

FIG. 29 is a view showing the deployment sequence with the first twelve links entering the stomach and tension being placed on the distal pull cable to pull it into position

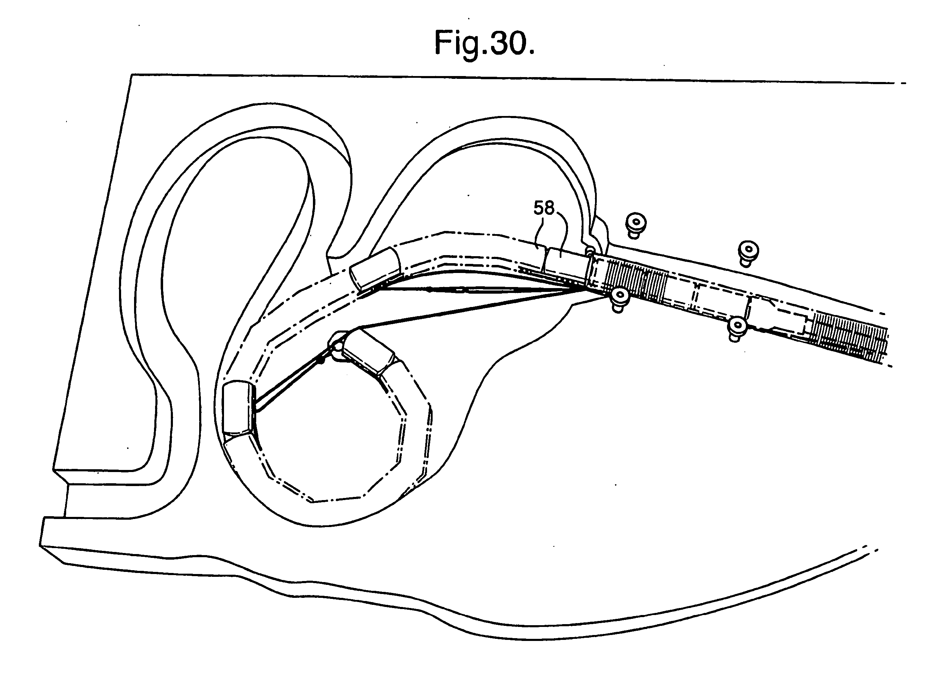

FIG. 30 is a view showing the deployment sequence with the first sixteen links entering the stomach and tension being placed on both pull cable's to pull them into position

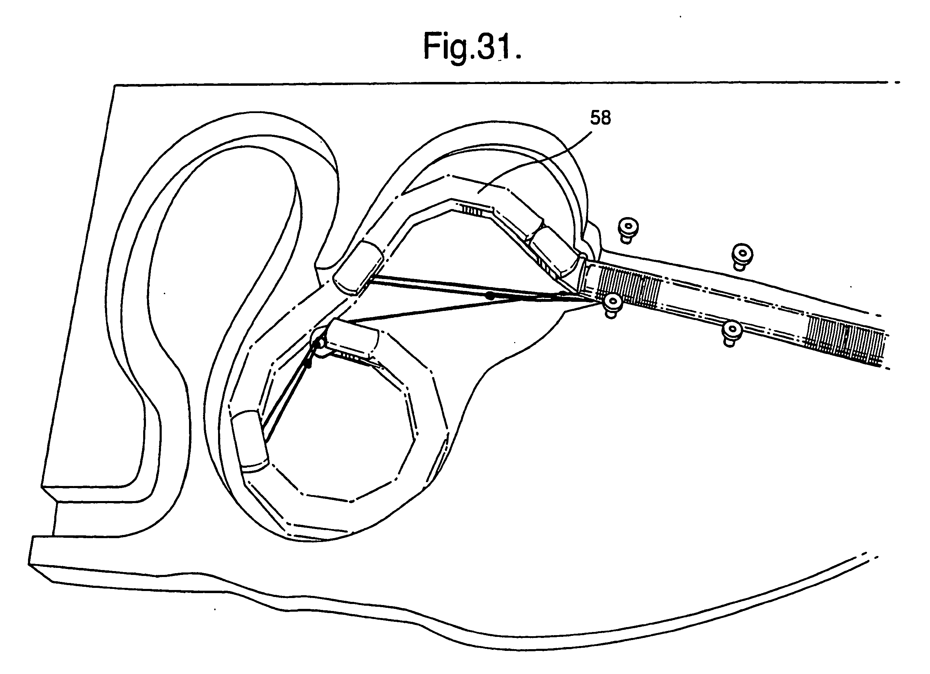

FIG. 31 is a view showing the deployment sequence with the first seventeen links entering the stomach and tension being placed on the proximal pull cable to hold it in position while the flexible endoscope advances the remaining coil into the stomach

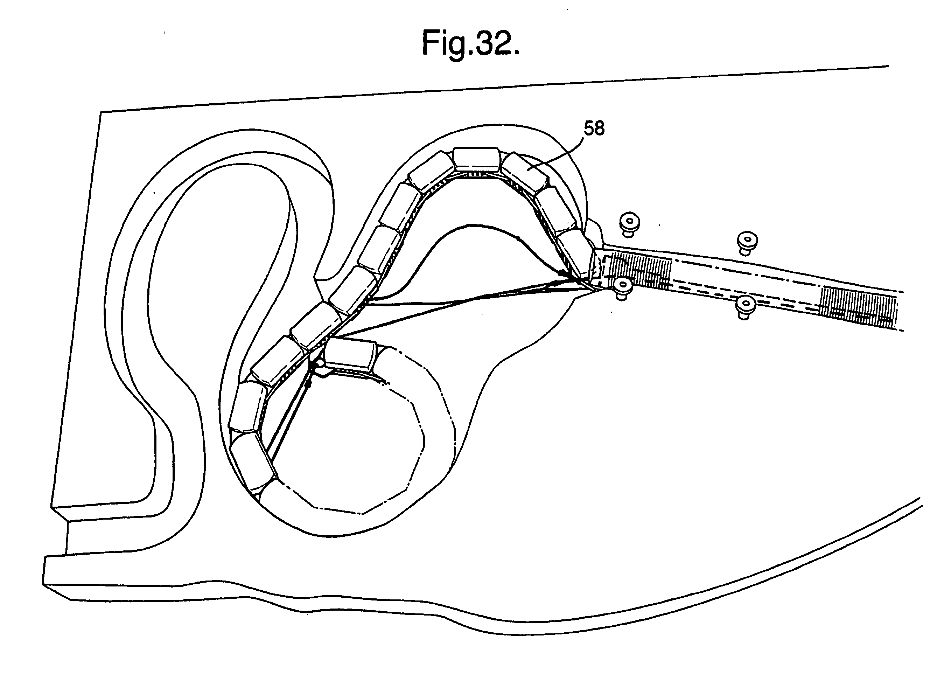

FIG. 32 is a view showing the deployment sequence with the entire coil entering the stomach.

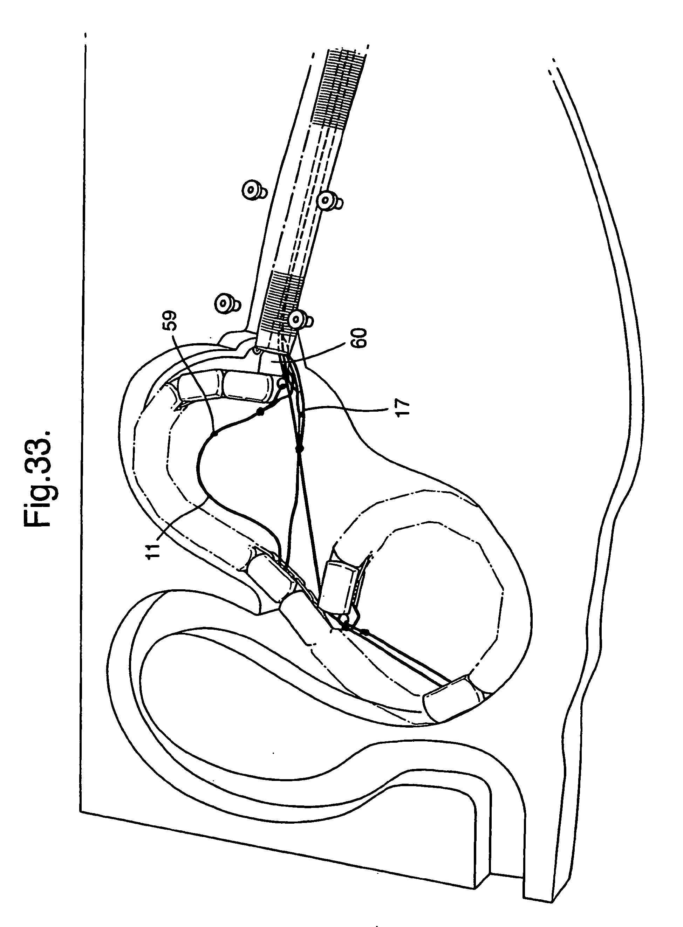

FIG. 33 is a view showing the deployment sequence with the entire coil entering the stomach

FIG. 34 is a view showing the deployment sequence with the entire coil deployed inside the stomach, the proximal string is being pulled to lock the proximal string lock into position.

FIG. 35 is a view showing the deployment sequence with the entire coil deployed inside the stomach, Both string lock's are locked into position.

FIG. 36 is a view showing the proximal pull cable being cut so the pull cable can be removed from the coil.

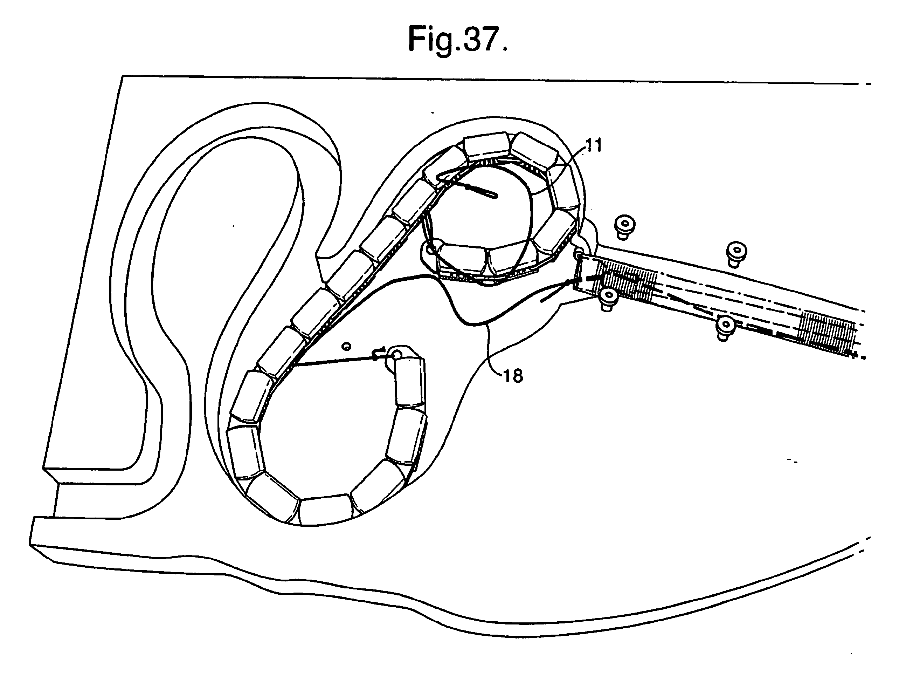

FIG. 37 is a view showing the resultant coil configuration after the proximal pull cable is cut and removed

FIG. 38 is a view showing the distal pull cable being cut so the pull cable can be removed from the coil.

FIG. 39 is a view showing the resultant coil configuration after the proximal and distal pull cable are both cut and removed

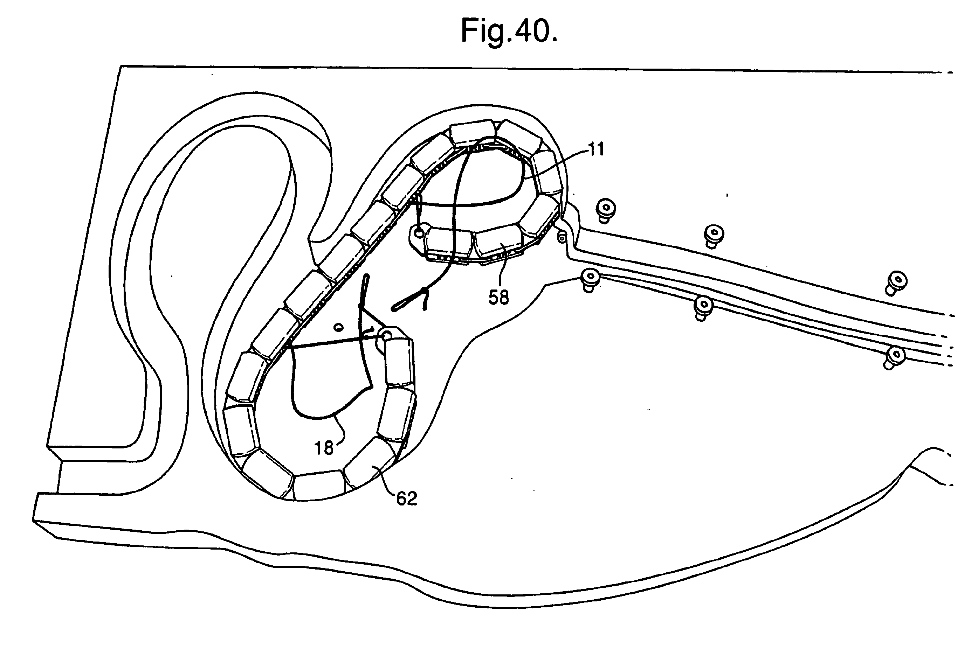

FIG. 40 is a view showing the resultant coil configuration after the cables, Flexible Endoscope, and Endoscopy Overtube are removed.

FIG. 41 is a isometric view showing the components of the string lock assembly

FIG. 42 is a isometric view showing the components of the string lock assembly

FIG. 43 is a isometric view showing the string lock component by itself

FIG. 44 is a top view showing the string routing with the knot prior to locking in the string lock component

FIG. 45 is a top view showing the string routing with the knot after locking in the string lock component

FIGS. 46 through 55 are deployment stages of the coil deployment inside the stomach

**DETAILED DESCRIPTION OF THE INVENTION**

The preferred embodiment of the Implantable Coil is made up of individual links made from HDPE injection molded plastic with a radio-opaque additive such as barium sulfate. The material must be Biocompatible to be implanted within the body. It may be made from a variety of materials including:
i. Polyetheretherketone (PEEK)
ii. High Density Polyethylene (HDPE)
iii. Polypropylene (PP)
iv. Low Density Polyethylene (LDPE)
v. Polysulfone (PSU)

The individual links are made up of a top link and a bottom link that are securely connected together with gripper pins where a hexagonal hole is made in the bottom link and a tapered pin 29 is made in the top link such that the tapered pin press fits into the hex hole 32 and securely holds the top and bottom together.

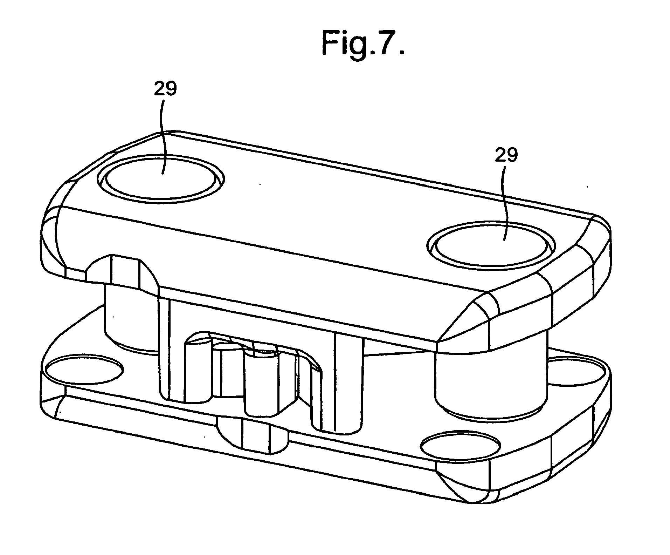

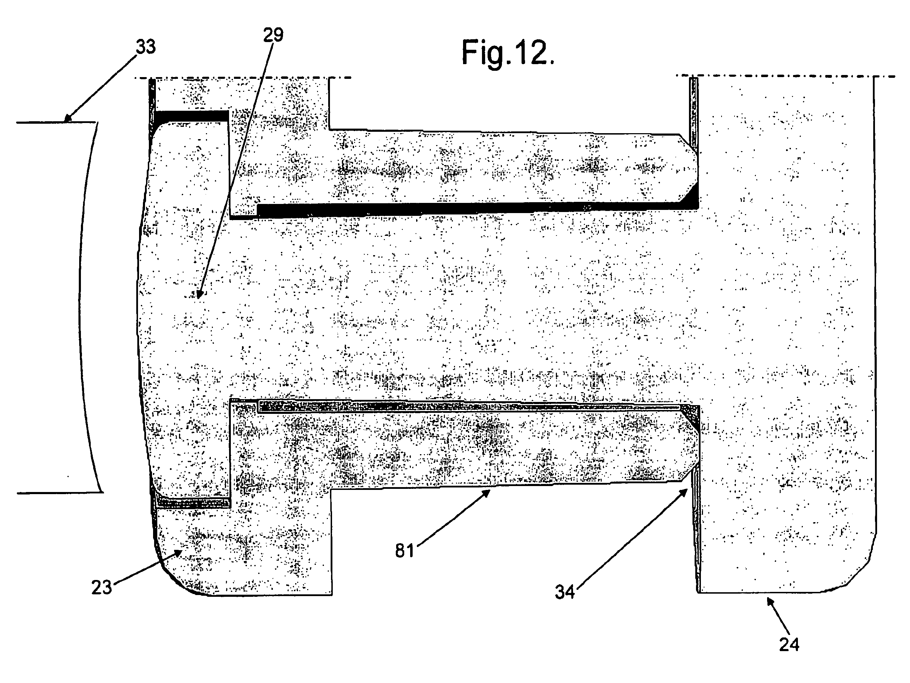

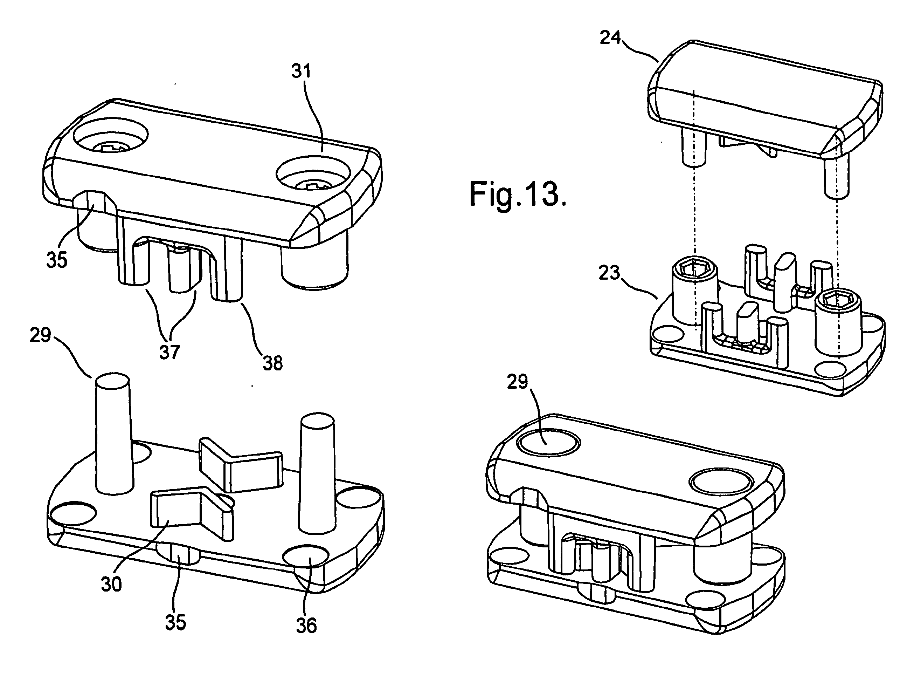

It may be necessary to heat stake the pin such that the pin is melted 29 into the hex hole to hold securely together as shown in Figure 7 and 12. The heat stake forming tool 33 is designed to provide the most heat transfer into the melted head of the boss 29 as shown in Fig 12, and Fig 13

A snap together fastener feature may alternately be used to hold these links together rather than the gripper pin design.

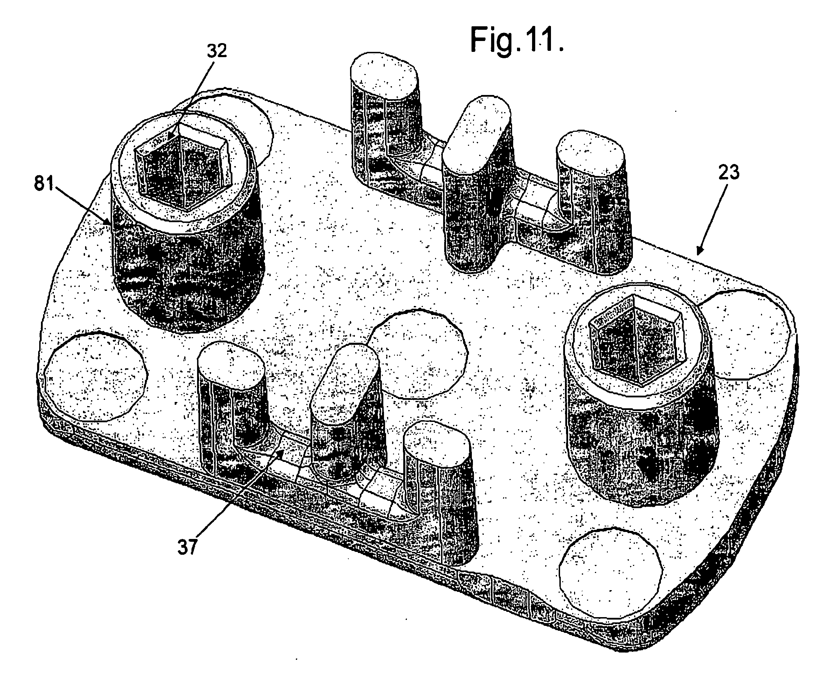

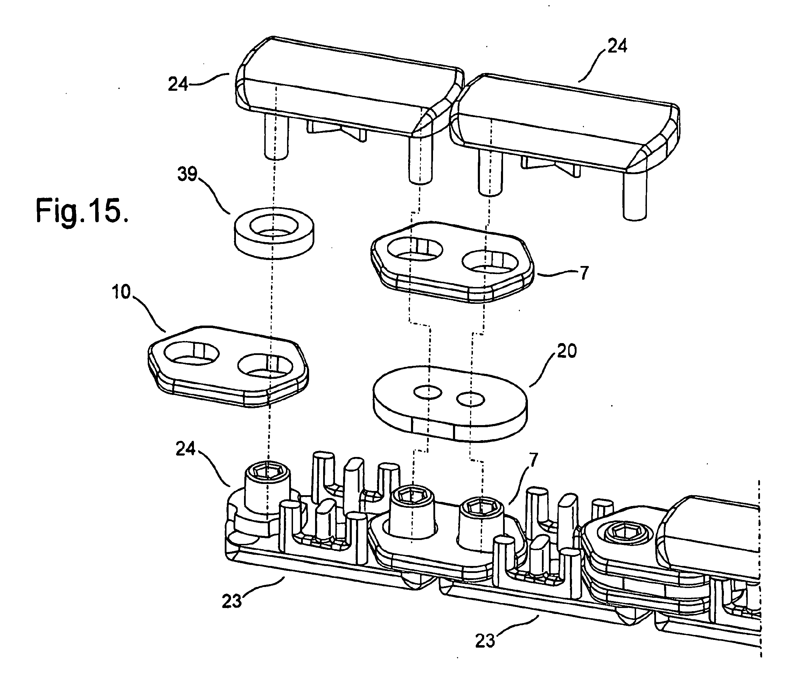

Fig 11, The outside diameter of the gripper pin feature 81 is also used as a pivot pin for the link connectors as shown in Fig. 15.

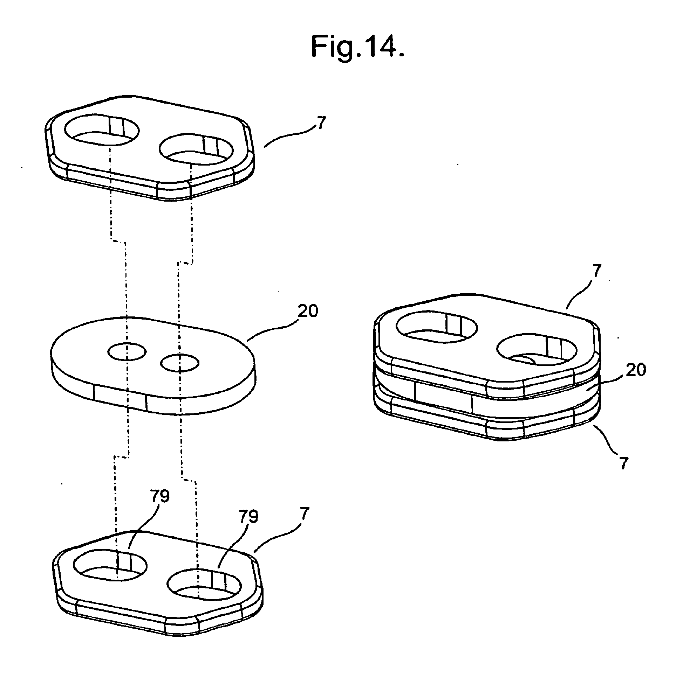

Fig. 14, The Link connector assembly is made up of a top link 7, and an elastomer link 20, and bottom link 7 on the bottom. This assembly is assembled over the pivot pin boss 81. The pivot pin boss 81 is symmetrical on each end of the link components 23,24.

The Link Components 23,24 can be injection molded in colors, The colors can be used to identify the proximal 58, center 55, and distal 62 sections of the Implantable Coil.

The Link components can also be individually numbered starting with the first being the distal most tip. And the second link and so on.

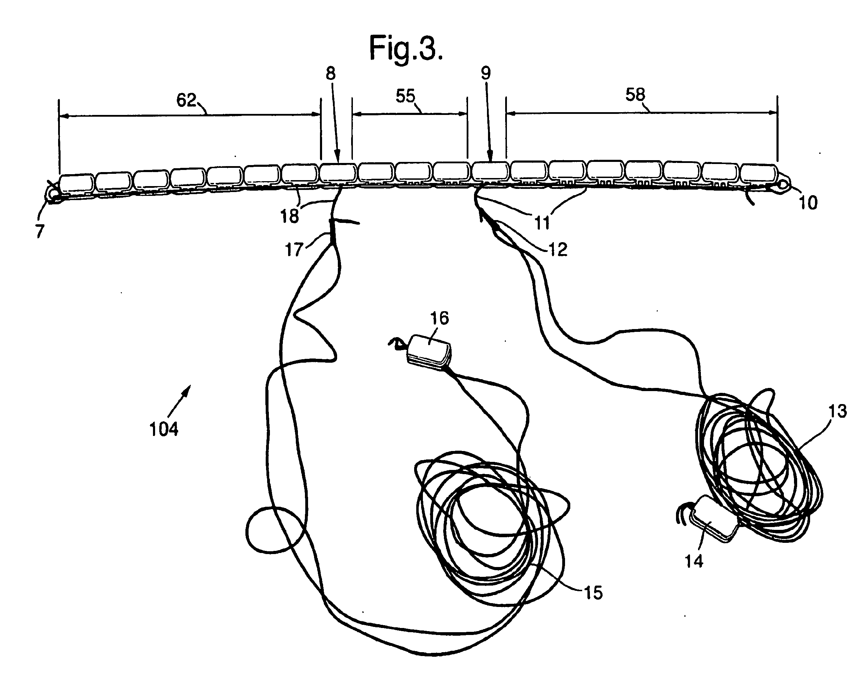

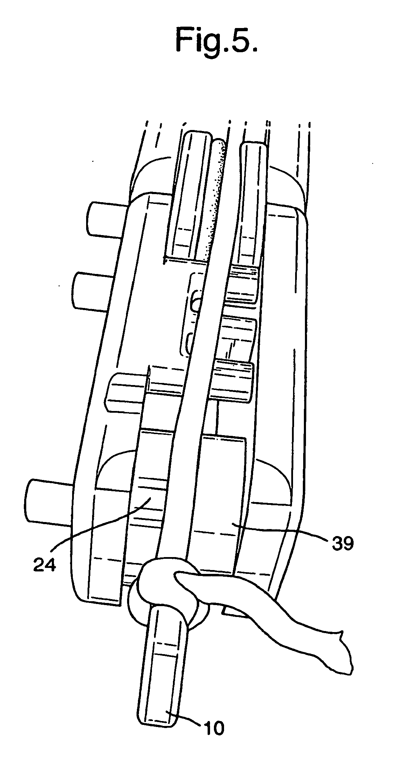

The Implantable Coil 104 further includes Radiopaque features that can be identified with Fluoroscopy Where the distal tip 7 of the Implantable coil Fig 3 is made up of two stainless steel spacers 78 that can easily be identified with a fluoroscope. The proximal tip 10 of the Implantable coil Fig 5 is made up of only one stainless steel spacers 24 that can easily be identified with a fluoroscope. This spacer 24 differs from the distal spacers 78 in that spacer 24 has scallops cut in the outer diameter that differintiate it from the distal end 7 while viewing with Fluoroscopy. The proximal tip 10 has a plastic spacer 39 that is not visible under Fluoroscopy. This combination of spacers provides a clear difference in the distal 7 and proximal 10 ends under Fluoroscopy.

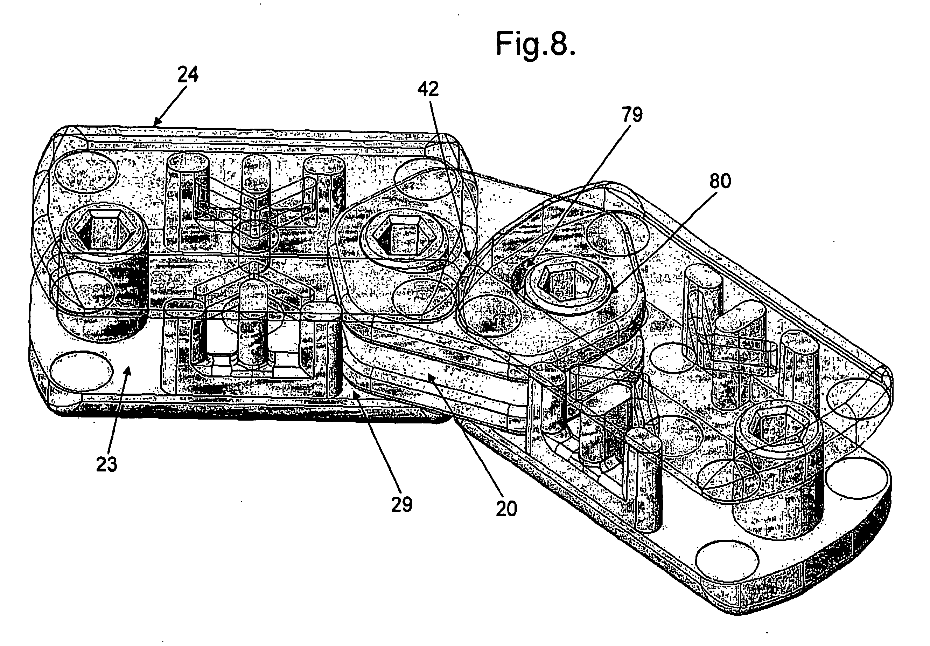

Fig. 8 The link joint can only bend to a pre-determined angle. This angle is determined by measuring in a pre-clinical environment, the minimum diameter that will pass from a hollow body organ into an adjoining lumen. Such as from the stomach into the pylorus. Once this diameter is determined, the device maximum angle was determined. The link connectors are designed to never bend beyond or smaller than this radius.

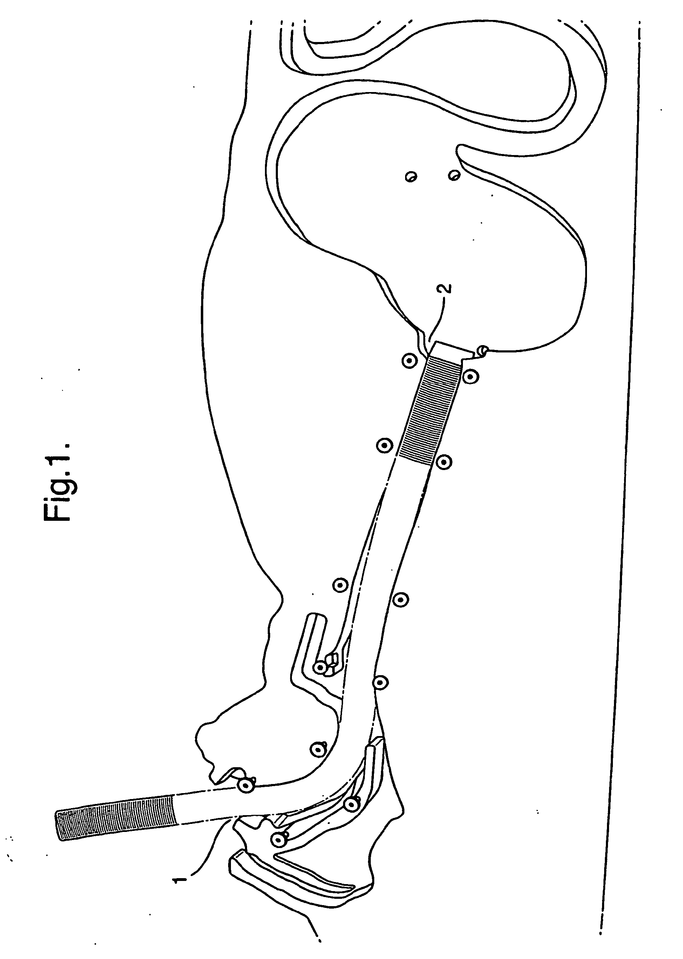

Bending of the Implantable Coil 104 in all directions is necessary as the coil must be able to pass down through the over tube. The over tube 3 makes some turns down through the mouth and into the esophagus 4 as shown in Fig. 1. The Coil is designed to flex easily in the direction as the coil bends around the pivot pins but in the perpendicular direction the tolerances of the parts must allow the link assembly to flex to get around these anatomical curves. To be flexible in this direction the total height of the Link connector assembly as shown in Fig 14. must be smaller than the

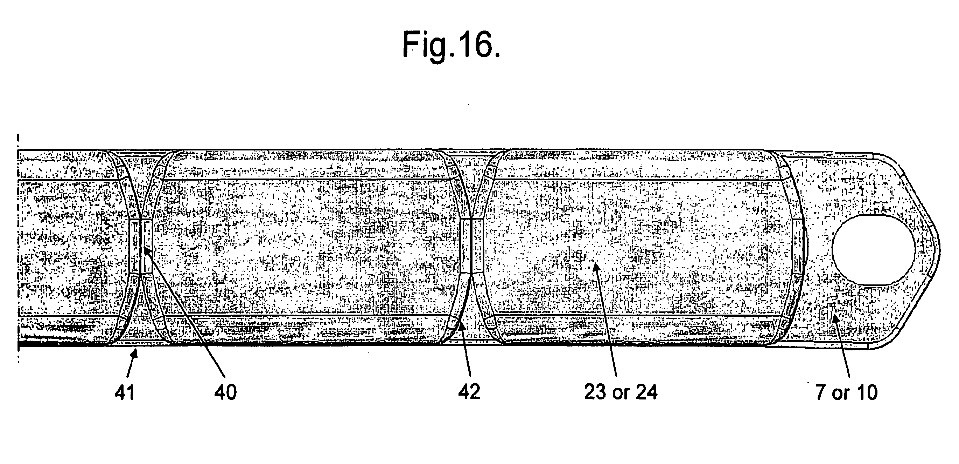

Fig. 16 The link component's 23, 24 has flat's 40 on each end. The Elastoner Link Connector 20 pulls the links 23, 24 together and causes these flat surfaces to come together. This has a self straightening effect on the assembly.

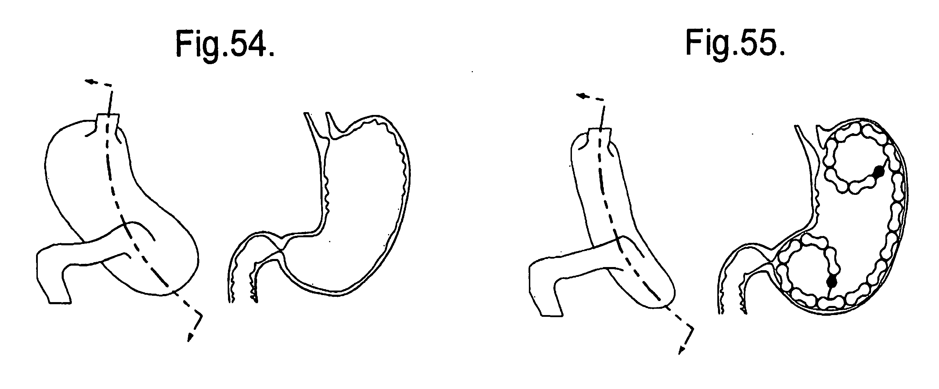

The Link connectors Fig 15 are also designed with an elastomer link connector 20, the elastomer link connector 20 pulls the links 23,24 together end to end 40 and allows the entire Implantable Coil to exert outward pressure on the hollow body organ Fig 55. This is accomplished by allowing the elastomer link connector 20 to flex/stretch which then allows the link to bend as shown in Fig 8. The rigid link connectors 7 have an oval hole 79 that acts as a positive stop at the pre-determined bending radius as shown in Fig. 8. The link component 23, 24 are bent into this configuration with a pull cable 11, 18. The positive stop bent configuration has several points that bottom out and prevent over bending of the link at 29, 42, and 80 as shown in Fig. 8. In this configuration Fig 8 the Elastomer Link Connector 20 is stretched to its maximum length. Fig 15, Note that the Elastomer Link Connector 20 must be stretched over the pivot pin boss 81 in order to be in a stretched position and to hold the flats 40 together as shown in Fig. 16.

The Link Connectors 7 provide a gap filling feature between the Link sections, as shown in Fig 8 Item 20 or in the straight configuration Fig. 16 Item 41 This overlap of components prevents tissue from getting between the Links, 23, 24

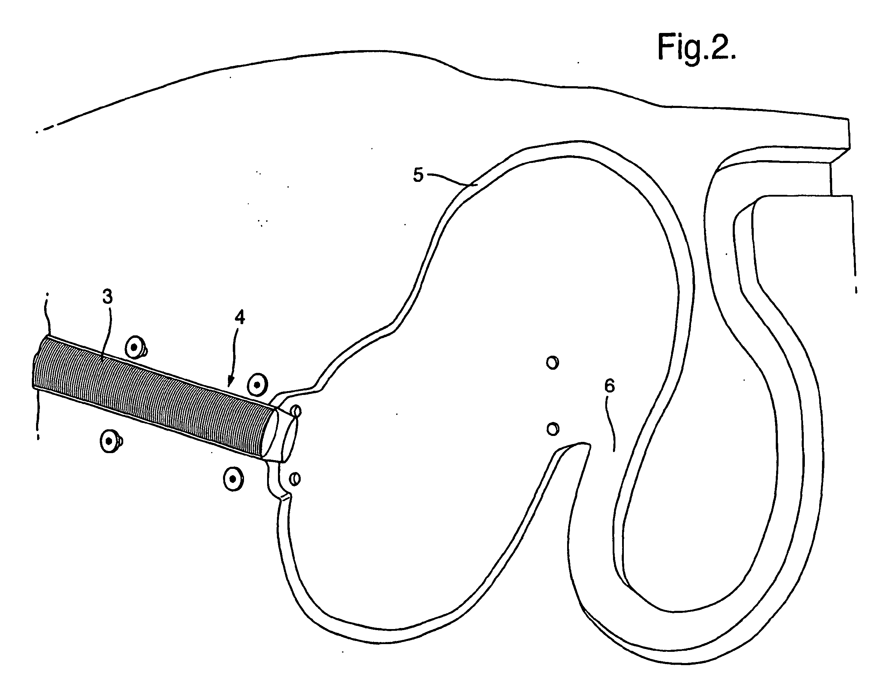

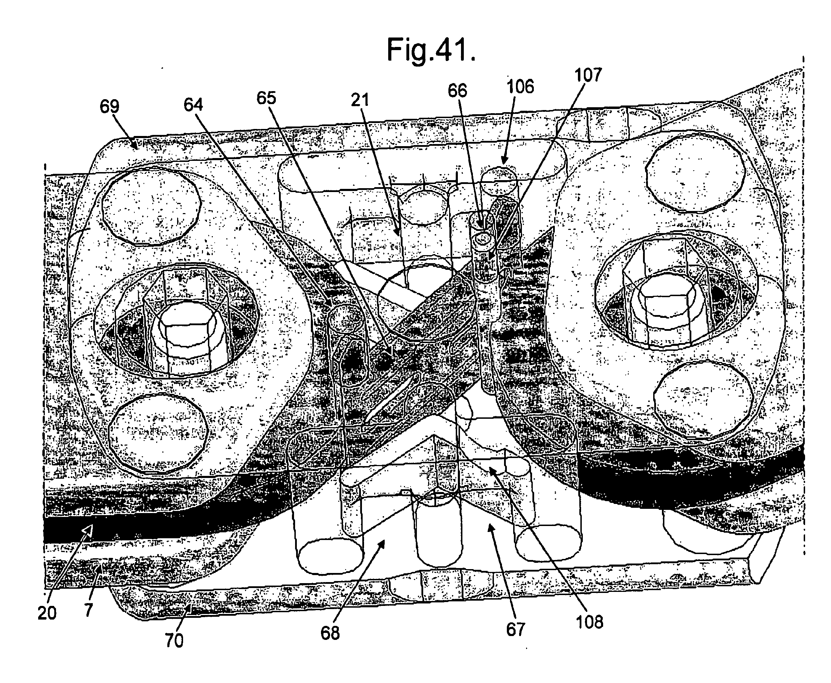

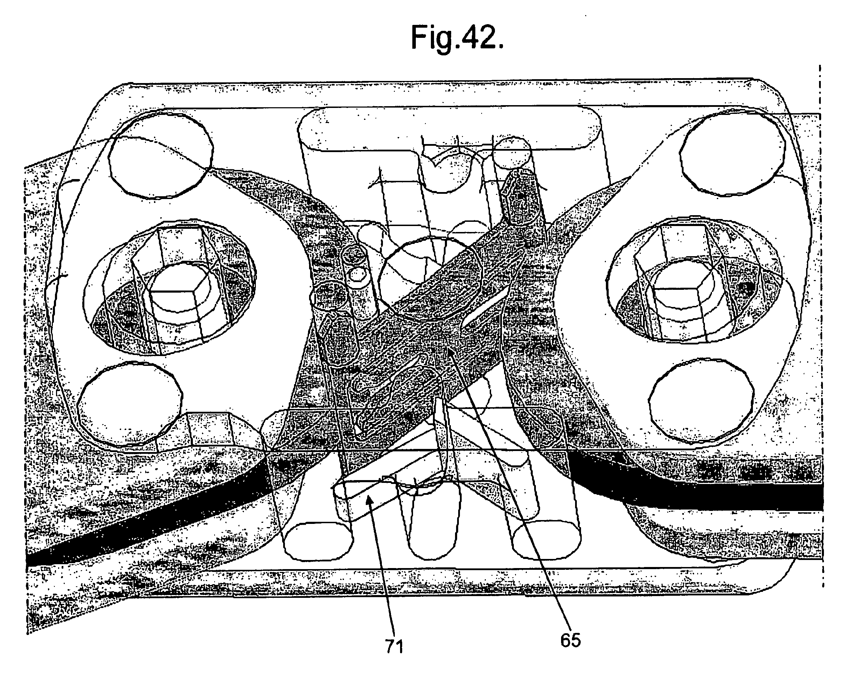

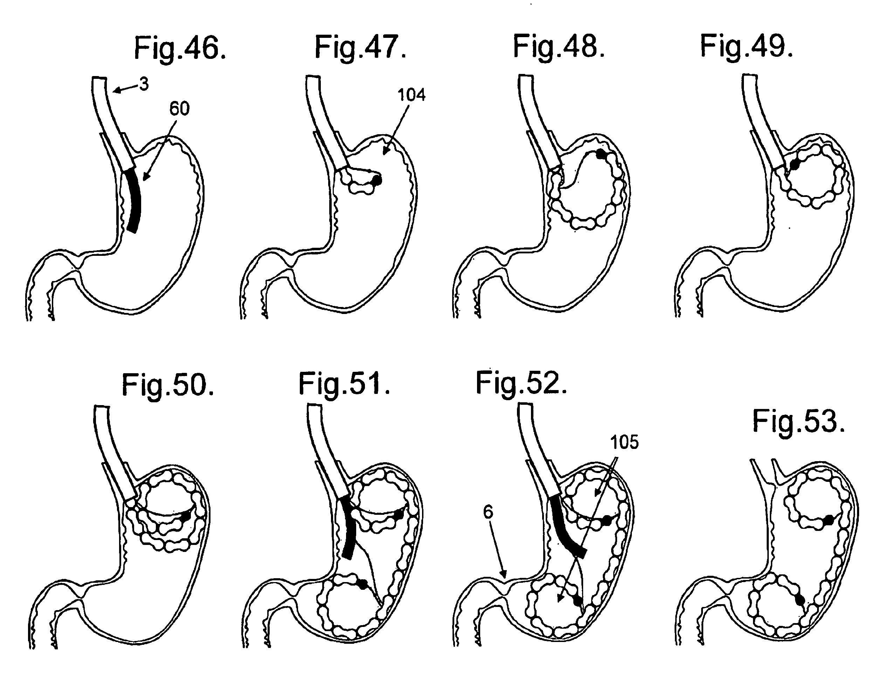

Fig. 3A String lock Link 8, 9 provides a locking means to allow the distal and proximal ends to be pulled into a curved diameter 105 as these curved ends will not allow passage of the Implantable Coil out of the hollow body organ into adjoining lumens such as the pylorus 6 as shown in Fig. 2. see Also Fig 52. The string lock link assembly is made up by a simple modification to the regular link components 23, 24. After hole's 64, 66, 106 are drilled through an existing parts 23, 24 they provide mounting for the string lock components. The new modified links are numbered in Fig. 41 as 69, and 70. The Link component 24 or now 70 with the male pin 29 also has a modification to the rib 30 on both sides. One side rib gets a small arc 108 milled out as shown in Fig. 41. The other rib 71 gets a portion of the rib milled off as shown in Fig 42. The main string lock components are the string lock 65, and the string lock pin 107. Both components are made from Biocompatible materials. They may be made from a variety of materials including: 316 Stainless Steel or suitable alternates.

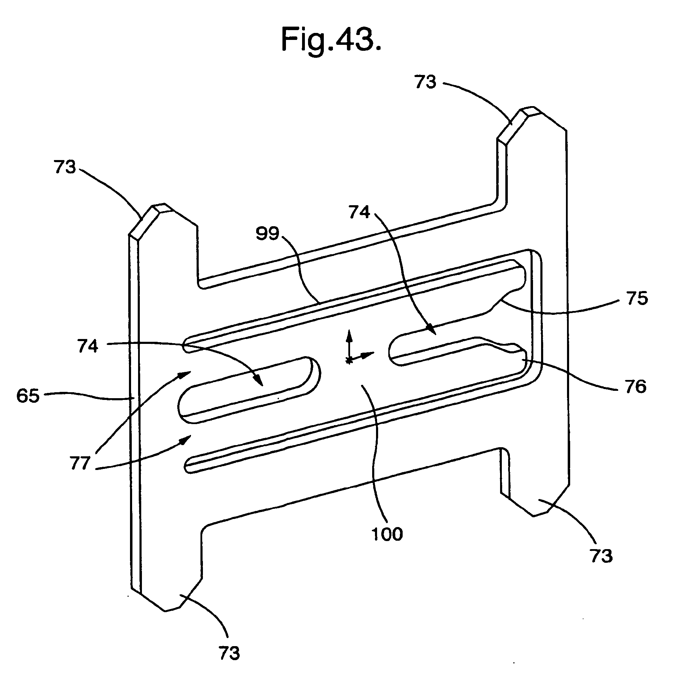

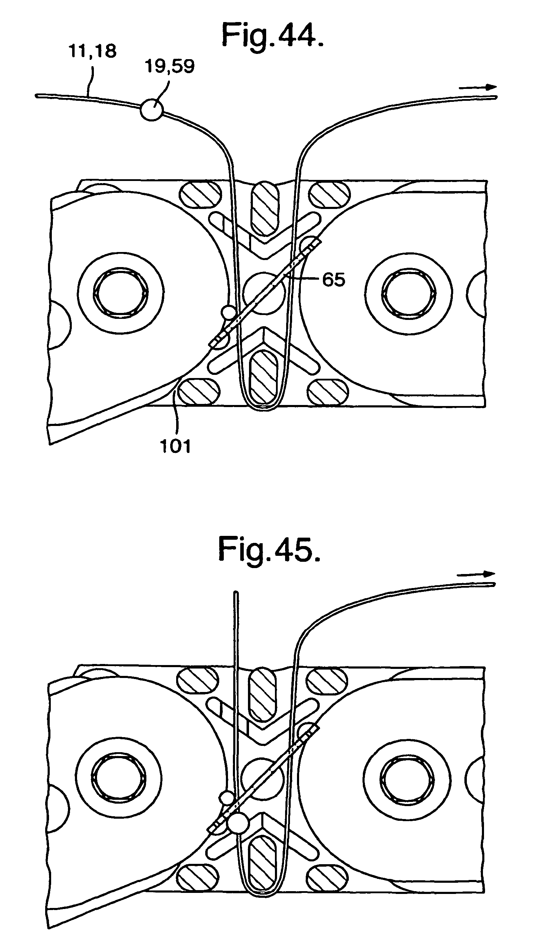

Fig. 43 The String Lock has a flexible arm 100 that is made from a single piece of Stainless Steel, A slot 99 is provided around the perimeter of the Flexible arm 100 to allow the arm to move as the string lock knot 19, 59 are pulled through during deployment. The arm flex's in area 77 while the post 73 are securely assembled in the holes 64, and 106 between the Modified Link Components 69 and 70. There are two slots 74 that provide free passage of the string lock cable 11, 18. Additional space 75 is provided in the tip area 76 of the flexible arm 100 for the knot 19 and 59 to snap over and lock into place holding the distal and proximal ends of the Implantable Coil into a curved diameter 105 as shown in Figures 44, 45, and 52.

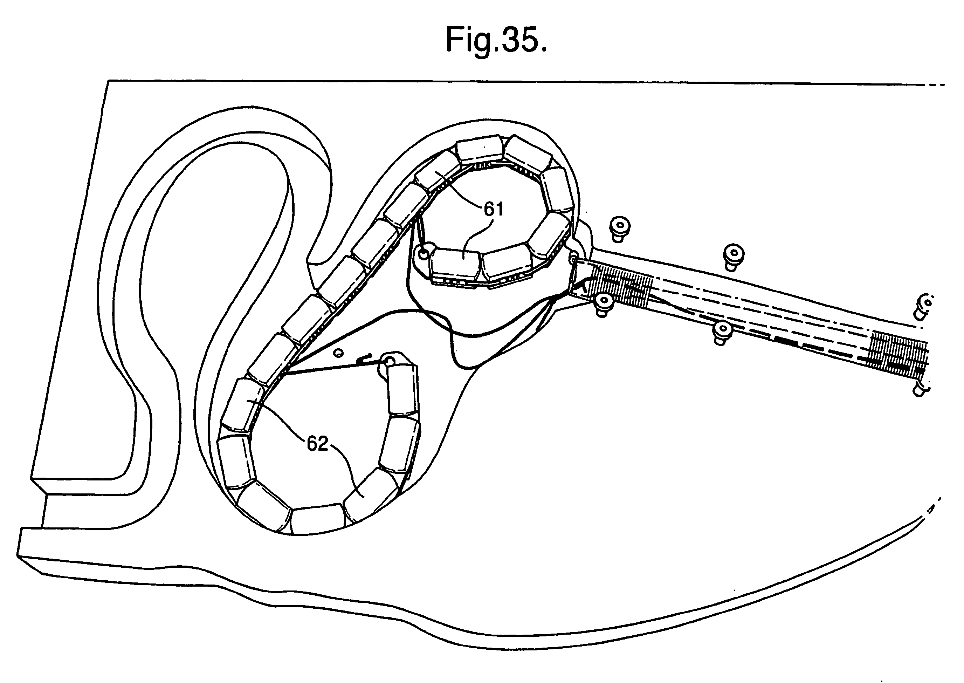

Fig. 3 Both ends of the Implantable Coil contain a loop of Removable pull cable 15 on the distal end and 13 on the proximal end. These pull cables consist of a long loop of cable connected at the ends with a Pull Link 16 at the distal end and 14 at the proximal end. The loop also is routed through a loop 17 distal and 12 proximal at the end of the string lock cables 18 distal and 11 proximal. After deployment is completed and the Implantable Coil is deployed as shown in Fig. 35 the Removable pull cable 15 on the distal end and 13 on the proximal can be cut near the Pull Link 16, and 14. One end of each cut loop can be pulled to remove the Removable pull cable 13, and 15 from the Implantable Coil assembly.

Fig. 46 Deployment of the over tube 3 in the esophagus 4 , inspect and assess the interior surfaces of the Stomach 5 with the endoscope 60 The preferred embodiment of the Implantable Coil 104 is to deploy it in the gastric cavity or stomach 5. The endoscope 60 is first advanced into the gastric cavity 5 to inspect and to orientate where the Implantable Coil 104 is to be placed. A guide wire may be used if necessary to re-insert the endoscope 60 with the over tube 3.

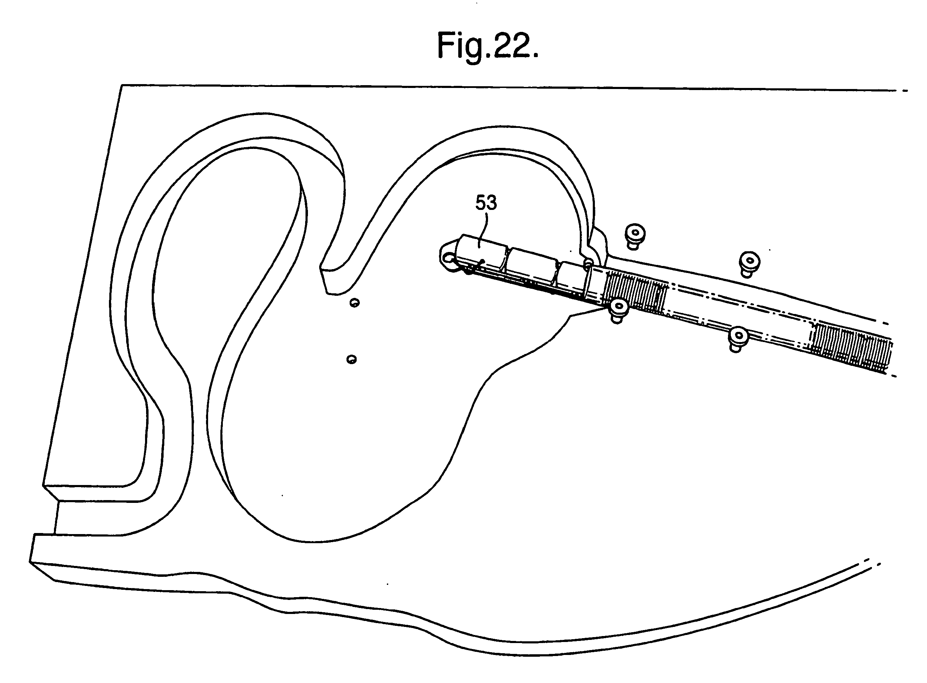

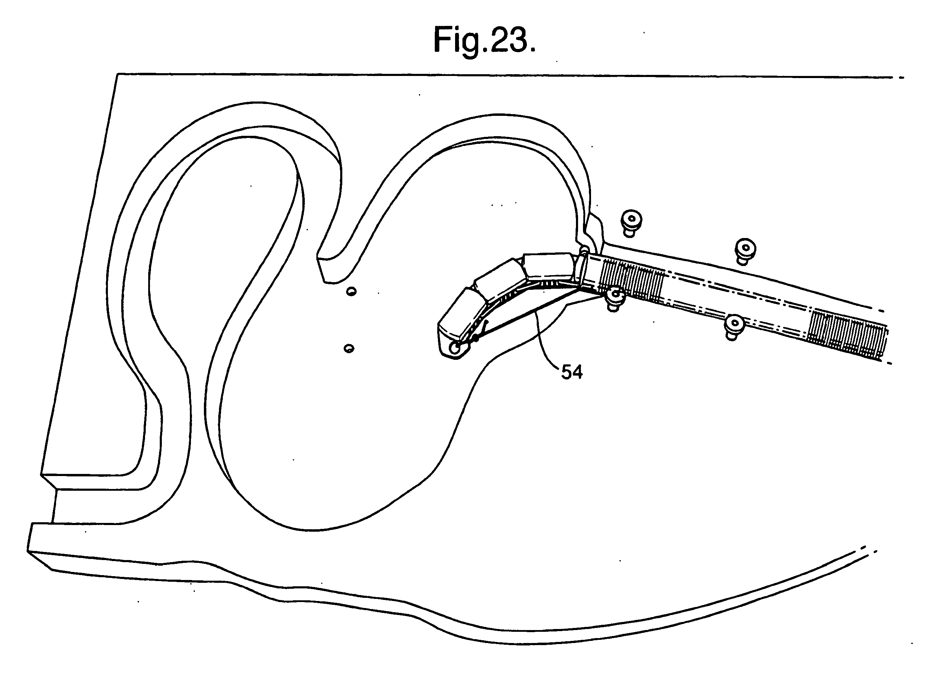

Fig. 47 Deployment of the distal portion of the Implantable Coil. Once the over tube 3 is placed as shown in Fig. 46 the Endoscope 60 is removed and the Implantable Coil 104 is inserted down the over tube 3. The Endoscope 60 is used to push the Implantable Coil 104 down the over tube 3 with a grasper through the working channel of the Endoscope 60. The grasper holds onto the Proximal tip 10 of the Implantable Coil 104. This assembly is then inserted down the over tube 3 First the distal tip 52 is ejected out the over tube tip inside the stomach as shown in Fig. 47. The Distal tip 53 continues its gentle deployment into the stomach as shown in Fig. 22. After 3 or 4 distal links are entered into the stomach, the distal pull cable 15 is pulled on from outside the body. The resultant distal cable 18 is pulled taunt 54 as shown in Fig. 23. As more Links 50 are gently advanced into the stomach the distal cable 18 continues to be pulled taunt 54 until the knot 19 is pulled inside the distal string lock link 8.

Fig. 48 Deployment of the distal portion of the Implantable Coil and Forming the distal portion of the coil into an arc The Knot 19 pulls through slot 74 and deflects flex arm 100 out of the path as the knot 19 is pulled through and locks on the other side of the flex arm 100 as shown in Fig's 43, 44, and 45

Fig. 49 Pull the distal pull cable 15 and lock the Distal string knot 19 into the distal string lock 8 Once locked in position the center coil section 55 continues to gently advance. The Distal Pull Cable 18 and 15 can be pulled while advancing to help make sure the distal tip deploys as needed in the gastric cavity.

Fig. 50 Advance the Center Links 55 into the stomach 5 Once locked in position the center coil section 55 continues to gently advance. The Distal Pull Cable 18 and 15 can be pulled to help make sure the distal tip moves in the gastric cavity as needed.

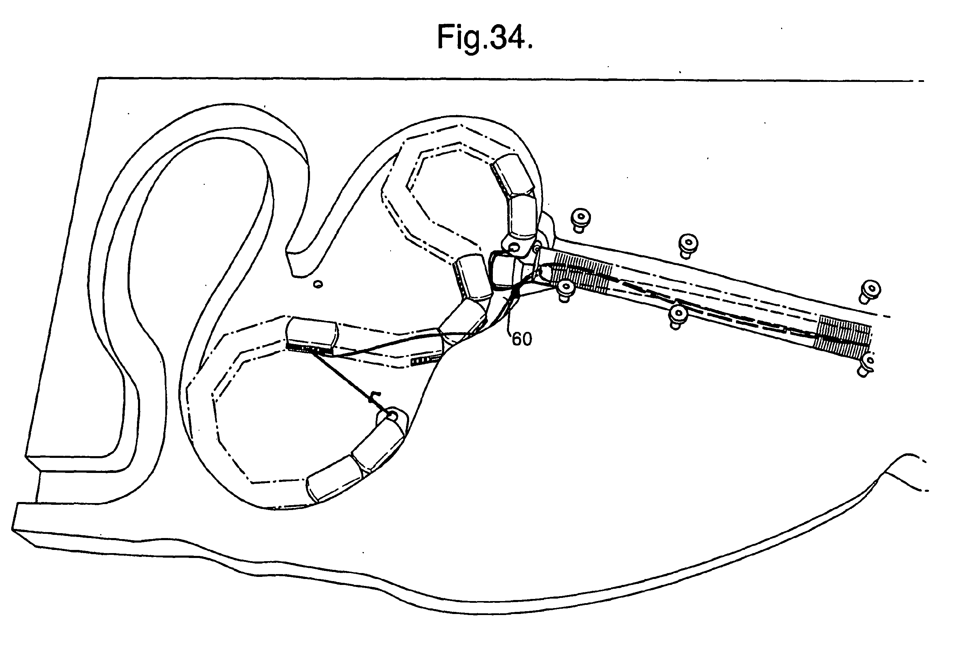

Fig 51 Advance the Proximal Links 58 into the stomach 5 and pull on the proximal pull cable 13 and lock the Proximal string knot 59 into the Proximal string lock 9 The Proximal End 58 continues advancing into the gastric cavity 5. First the Proximal Links 58 are advanced while gently pulling on the Proximal Pull Cable 11, and 13. This brings the proximal Links 58 into the gastric cavity 5 in a arc where the links push outward along the greater curve. This allows all of the Proximal Links 58 to be deployed inside the stomach 5. The Proximal Pull Cable 11, and 13 are then pulled to lock the Proximal String Lock 9 which temporarily pulls the links away from the greater curve of the stomach as shown in Fig. 34.

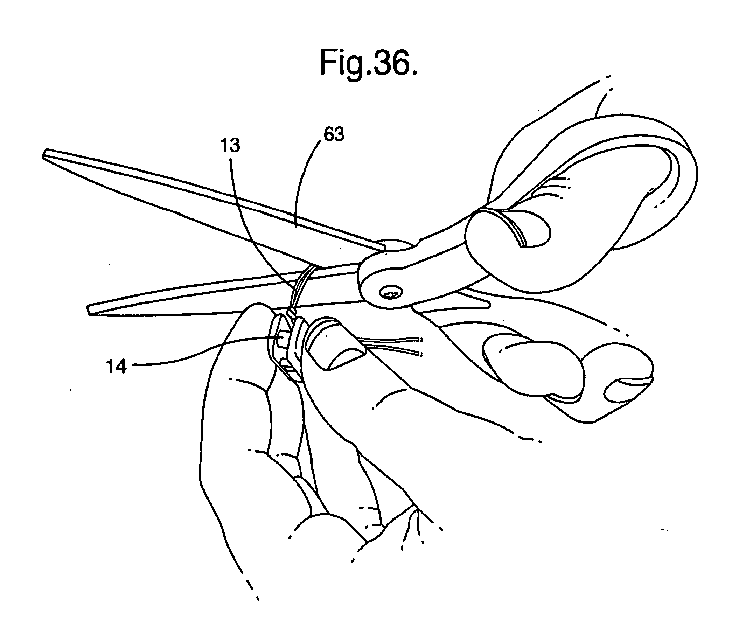

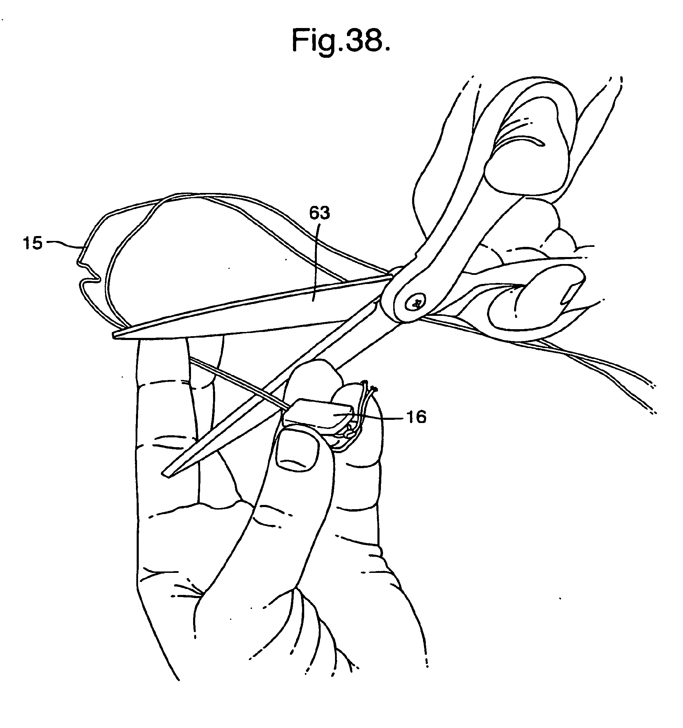

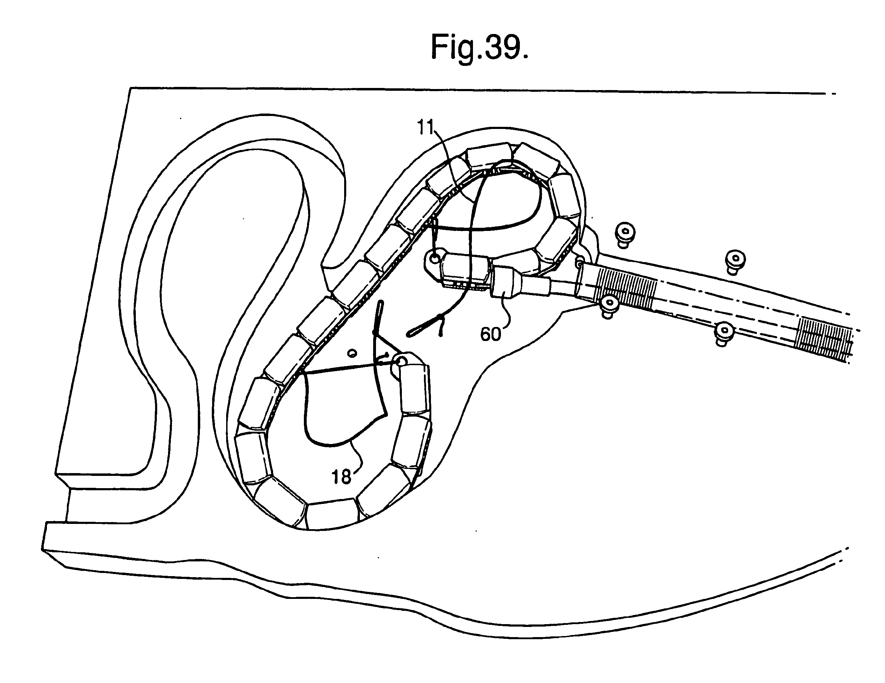

Fig. 52 Inspect placement of the Implantable Coil 104 with endoscope 60 The final step prior to cutting the Pull Cable 11, and 13 is to release the graspers that were used to hold the proximal tip 10. The graspers are used through the Endoscope working channel to hold the proximal tip 10 to advance it down the over tube 3. To remove the removable portion of the pull cable 13, and 15 a pair of scissors 63 are used to cut the cables near the pull block 14, and 16 as shown in Fig. 36, and 38. A final inspection can be done with the Endoscope 60 after the removal of the removable pull cables 13 and 15 as shown in Fig 39.

Fig. 53 Remove the Removable Pull Cables 13, 15, Remove the Over Tube 3, and Endoscope 60

Fig. 54 This figure shows what a stomach looks like from both a side and front view before the Implantable Coil 104 is implanted.

Fig. 55 This figure is the same as Fig. 54 except that the Implantable Coil 104 is implanted and the stomach is distended in the front plane where it brings the anterior and posterior surfaces together.

## Claims

1. An implant for placement within a hollow body organ (55), said implant comprising:
a. a member (140) having an undeployed shape for delivery to the hollow body (55) and a deployed shape for implantation therein;
b. said member (140) comprising a plurality of links (23,24) pivotably connected to each other, said implant further comprising a flexible elongated tether (20) connected to said member such that tensioning said tether places said member in said deployed shape;
c. said member (140) having sufficient rigidity in its deployed shape to exert an outward force against an interior of the hollow body (55) so as to bring together two substantially opposing surfaces of said hollow body (55); and **characterized by**
d. an elastomer link connector (20) configured to pull the links together.

## Patentansprüche

1. Implantat zum Platzieren in einem Hohlkörperorgan (55), wobei das Implantat Folgendes umfasst:
a. ein Element (140) mit einer nicht entfalteten Gestalt zum Ausbringen an den Hohlkörper (55) und einer entfalteten Gestalt zur Implantation darin,
b. wobei das Element (140) mehrere Verbindungsglieder (23, 24) umfasst, die schwenkbar miteinander verbunden sind, wobei das Implantat ferner ein flexibles längliches Halteseil (20) umfasst, das so mit dem Element verbunden ist, dass das Element durch das Spannen des Halteseils in die entfaltete Gestalt gebracht wird,
c. wobei das Element (140) in seiner entfalteten Gestalt ausreichend starr ist, um gegen einen Innenraum des Hohlkörpers (55) eine äußere Kraft auszuüben, um zwei sich im Wesentlichen gegenüberliegende Flächen des Hohlkörpers (55) zusammenzubringen, **gekennzeichnet durch**
d. einen Elastomerverbindungsgliedverbinder (20), der so konfiguriert ist, dass er die Verbindungsglieder zusammenzieht.

## Revendications

1. Implant destiné à être placé dans un organe corporel creux (55), ledit implant comprenant :
a. un organe (140) ayant une forme non déployée pour son introduction dans le corps creux (55) et une forme déployée pour son implantation dans celui-ci ;
b. ledit organe (140) comprenant une pluralité de liaisons (23, 24) connectées de manière pivotante les unes aux autres, ledit implant comprenant en outre un tirant flexible allongé (20) connecté audit organe de telle sorte que le tensionnement dudit tirant amène ledit organe à ladite forme déployée ;
c. ledit organe (140) ayant une rigidité suffisante dans sa forme déployée pour exercer une force extérieure contre un intérieur du corps creux (55) de manière à amener l'une contre l'autre deux surfaces substantiellement opposées dudit corps creux (55) ; et **caractérisé par**
d. un connecteur de liaison élastomère (20) configuré pour attirer les liaisons l'une contre l'autre.
